# EUROPEAN PATENT SPECIFICATION

(11) **EP 4 475 834 B1**
(45) Date of publication and mention of the grant of the patent: **09.12.2026**
(21) Application number: 23703446.7
(22) Date of filing: 07.02.2023
(51) Int. Cl.: A61K 31/215, A61P 31/04

(54) **LEFAMULIN AND ITS DERIVATIVES FOR USE IN THE TREATMENT OF A SPIRAL SHAPED BACTERIA**
LEFAMULIN UND DESSEN DERIVATE ZUR VERWENDUNG BEI DER BEHANDLUNG VON SPIRALFÖRMIGEM BAKTERIUM
LEFAMULIN ET SES DÉRIVÉS POUR LEUR UTILISATION DANS LE TRAITEMENT D'UNE BACTÉRIE EN FORME DE SPIRALE

(30) Priority: 09.02.2022 US 202263308163 P; 31.03.2022 EP 22166177
(43) Date of publication of application: 18.12.2024
(73) Proprietor: Hong Kong King-Friend Industrial Company Limited, Hong Kong (HK)
(72) Inventor: GASSNER, Gisela, 3304 St. Georgen am Ybbsfelde (AT); GELONE, Steven, Flourtown, Pennsylvania 19031 (US); PAUKNER, Susanne, 1160 Wien (AT); WICHA, Wolfgang, 2460 Bruck a. d. Leitha (AT)
(74) Representative: Schwarz & Partner Patentanwälte GmbH
(86) International application number: PCT/EP2023/052949
(87) International publication number: WO 2023/152115

(56) References cited:
- WO-A1-2008/113089
- WO-A1-2009/106839
- WO-A1-2022/219182
- CN-A- 111 170 911
- VEVE MICHAEL P. ET AL: "Lefamulin: Review of a Promising Novel Pleuromutilin Antibiotic", vol. 38, no. 9, 20 August 2018 (2018-08-20), US, pages 935 - 946, XP055946676, ISSN: 0277-0008, Retrieved from the Internet <URL:https://api.wiley.com/onlinelibrary/tdm/v1/articles/10.1002%2Fphar.2166> DOI: 10.1002/phar.2166
- KITAI KAZUHISA ET AL: "In Vitro Activity of 39 Antimicrobial Agents Against Treponema hyodysenteriae", ANTIMICROBIAL AGENTS AND CHEMOTHERAPY, vol. 15, no. 3, 1 March 1979 (1979-03-01), US, pages 392 - 395, XP055946685, ISSN: 0066-4804, DOI: 10.1128/AAC.15.3.392

## Description

The present invention relates to pleuromutilin derivatives of formula (I), such as lefamulin, for use in the treatment or prevention of a bacterial infection mediated by spirochetes.

Pleuromutilin, a compound of formula (A) 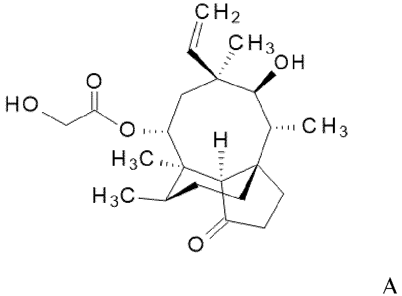 is a naturally occurring antibiotic, produced e.g. by the basidiomycetes *Pleurotus mutilus* and *P. passeckerianus,* see e.g. The Merck Index, 12th edition, item 7694.

A number of further Pleuromutilins having the principle ring structure of Pleuromutilin and being substituted at the primary hydroxy group have been developed, e.g. as antibacterials. Due to their pronounced antibacterial activity, a group of Pleuromutilin derivatives, amino-hydroxy-substituted cyclohexylsulfanylacetylmutilins, as disclosed in WO 2008/113089, have been found to be of particular interest. As described in WO 2008/113089 14-O-{[(4-Amino-2-hydroxy-cyclohexyl)-sulfanyl]-acetyl}-mutilins are particularly useful compounds because of their activity against Gram-positive and Gram-negative bacteria.

Pharmaceutical active compounds derived from Pleuromutilin (semi synthetic compounds) are inhibitors of ribosomal protein synthesis in bacteria. Representatives of semisynthetic Pleuromutilins for human use are Retapamulin (compound of formula (B), approved as AltargoP^{®}, AltabaxP^{®}), a topical agent approved for short term treatment of impetigo and infected small lacerations, abrasions or sutured wounds, and Lefamulin (compound of formula (VII), approved as Xenleta^{®}) for the treatment of adults with community-acquired bacterial pneumonia (CABP). Valnemulin (compound of formula (C), approved as Econor^{®}) and Tiamulin (compound of formula (D), approved as Denagard^{®}) are two other semi-synthetic Pleuromutilin derivatives which have been used systemically as antibiotics in veterinary medicine for many years.

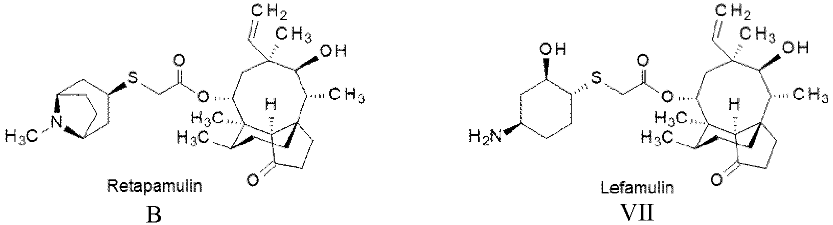

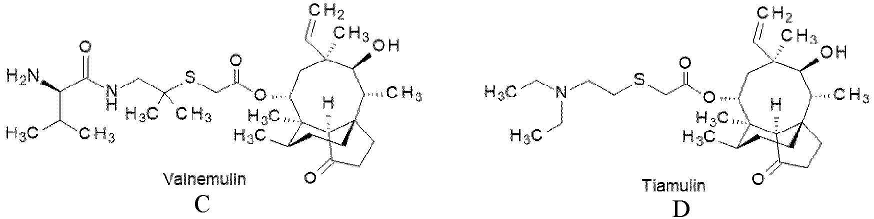

Approved semisynthetic compounds derived from Pleuromutilin have shown excellent activity against bacterial organisms which include inter alia *Streptococcus pneumoniae, Haemophilus influenzae, Staphylococcus aureus* (including MRSA), *Moraxella catarrhalis, Legionella pneumophila, Chlamydophila pneumoniae* and *Mycoplasma pneumoniae.*

Tiamulin is used for the treatment of swine dysentery caused by or further complicated by tiamulin-susceptible *Brachyspira hyodysenteriae.* The activity of tiamulin (81723 hfu) against the causative agent of swine dysentery, formerly also referred to as *Treponema hyodynsenteriae,* was reported back in 1976 (Laber, G. Activity of Various Compounds Against a Pathogenic Strain of Treponema hyodynsenteriae. Zentralbl Bakteriol Orig A. 236, 127-130 (1976). Sandoz Forschungsinstitut Wien; Kitai, K., Kashiwazaki, M., Adachi, Y. et al. In Vitro Activity of 39 Antimicrobial Agents Against Treponema hyodysenteriae. Antimicrob Agents Chemother 15, 392-396 (1979) doi: 10.1128/AAC.15.3.392). This spirochete bacteria remains very sensitive to tiamulin although resistance against*B*. *hyodysenteriae* has been reported (Huvepharma NV, Summary of Product Characteristics Vetmulin 125 mg/ml solution, http://mri.cts-mrp.eu/download/FR_V_0202_001_FinalSPC.pdf, link accessed on February 01, 2022).

Also Valnemulin hydrochloride is approved as veterinary medicinal product Econor^{®} for medicated feed for the target species pigs and rabbits. The indications cover treatment and prevention of swine dysentery. Valnemulin shows high activity *against Mycoplasma spp.* and *Lawsonia intracellularis* as well as the spirochaetes *Brachyspira hyodysenteriae* and *Brachyspira pilosicoli.* (Elanco GmbH, Summary of Product Characteristics, Econor 10 % and 50%, https://ec.europa.eu/health/documents/community-register/2020/20200805148861/anx_148861_en.pdf, link accessed on February 01, 2022).

A high-throughput screening of the NCI-DTP Library inter alia revealed Pleuromutilin (NCI 121145) as initial hit compound, but minimum inhibitory (MIC) and minimum bactericidal concentration (MBC) less promising than other hits (Pothineni, V., Wagh, D., Babar, M. et al. Screening of NCI-DTP library to identify new drug candidates for Borrelia burgdorferi. J Antibiot 70, 308-312 (2017). doi: 10.1038/ja.2016.131)

Lefamulin was also identified to have promising activity against bacteria mediating sexually transmitted diseases (STDs) (Jacobsson, S., Paukner, S., Golparian, D., Jensen, J.S., Unemo, M. In vitro activity of the novel pleuromutilin lefamulin (BC-3781) and effect of efflux pump inactivation on multidrug-resistant and extensively drug-resistant Neisseria gonorrhoeae. Antimicrob Agents Chemother 61:e01497-17 (2017). doi: 10.1128/AAC.01497-17; Paukner, S., Gruss, A., Jensen, J.S., In vitro activity of lefamulin against sexually transmitted bacterial pathogens. Antimicrob Agents Chemother 62:e02380-17 (2018). doi: 10.1128/AAC.02380-17). The *in vitro* activity includes C. *trachomatis,* gonococci, mycoplasmas, ureaplasmas and anaerobic cocci causing gonorrhea, non-gonococcal urethritis, cervicitis and pelvic inflammatory disease.

A workshop by US FDA on "Development Considerations of Antimicrobial Drugs for the Treatment of Gonorrhea" was held on April 23, 2021. The slides of a talk entitled *"Development Considerations for a Syndromic Approach to Uncomplicated Urethritis*/*Cervicitis"* as well as a transcript of the workshop were thereafter made available for downloaded from the FDA webpage (https://www.fda.gov/drugs/news-events-human-drugs/development-considerations-antimicrobial-drugs-treatment-gonorrhea-04232021-04232021, with content of May 26, 2021) , in particular from https://www.fda.gov/media/149520/download and https://www.fda.gov/media/148225/download (both links accessed on February 01, 2022).

### SUMMARY OF THE INVENTION

Surprisingly, it has now been found that the Pleuromutilin derivatives disclosed in WO 2008/113089A1 show antimicrobial activity against several spiral bacteria in particular being spirochetes, more precisely bacteria selected from the phylum of Spirochaetes. The Pleuromutilin derivatives are active *in vitro* and *in vivo* against bacteria known to cause severe bacterial infections in humans.

Therefore, in a first aspect, the present invention relates to a compound as defined in claims 1 to 6, in particular Lefamulin, or any pharmaceutically acceptable salt, solvate thereof, for the specific use in the treatment or prevention of a bacterial infection mediated by spirochetes or by bacteria selected from the phylum of Spirochaetes.

### BRIEF DESCRIPTION OF THE DRAWINGS

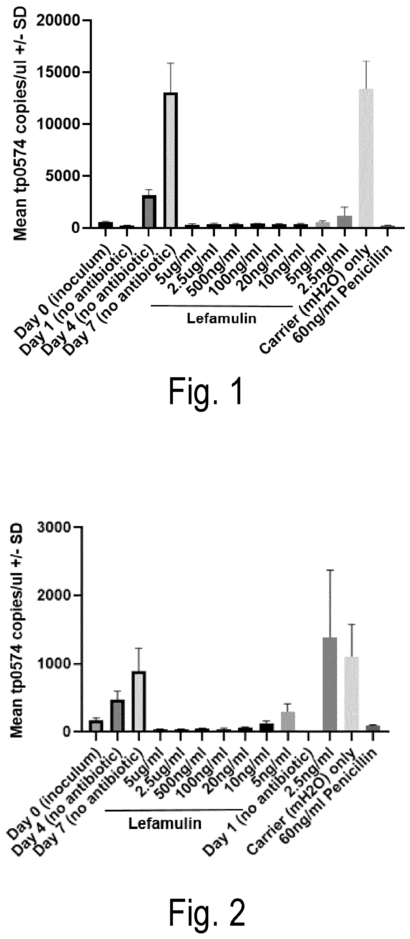

Figure 1 is a graph showing the dose-dependent antibacterial effect of an exemplary compound of the present invention, Lefamulin, against *T. pallidum* in an *in vitro* model, wherein the antibacterial activity against *T. pallidum* Chicago strain is reflected in a reduction of the gene copy numbers of the *T. pallidum* gene *tp0574* per µL as determined by quantitative real-time PCR compared to different no antibiotic controls and to penicillin G (60 ng/mL) as positive control.
Figure 2 is a graph showing the dose-dependent antibacterial effect of an exemplary compound of the present invention, Lefamulin, against *T. pallidum* in an *in vitro* model, wherein the antibacterial activity against *T. pallidum* SS14 strain is reflected in a reduction of the gene copy numbers of the *T. pallidum* gene *tp0574* per µL as determined by quantitative real-time PCR compared to different controls (no antibiotic) and to penicillin G (60 ng/mL) as positive control.

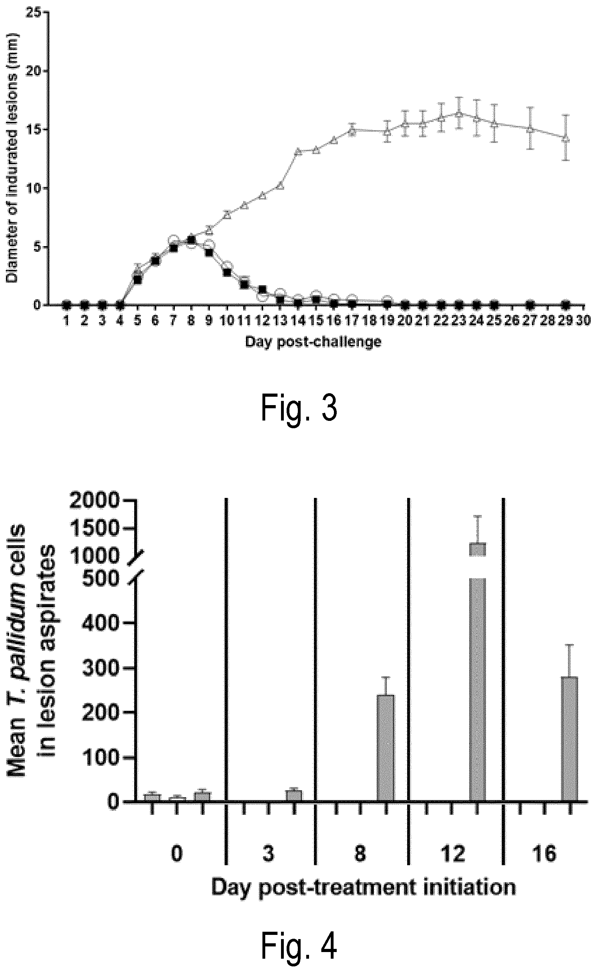

Figure 3 is a graph showing the effect of an exemplary compound of the present invention, Lefamulin, on size (diameter) of the lesions in an *in vivo* Syphilis model over time, wherein the diameter of indurated lesions at the site of intradermal infection with *T. pallidum* Chicago strain was investigated in three groups of infected rabbits (each group n=2-3 animals) covering a Lefamulin-treated group (open circles), a control group treated with Benzathine penicillin G (squares), and an untreated group (open triangles).
Figure 4 is a graph showing the effect of an exemplary compound of the present invention, Lefamulin, on the treponemal burden within the lesions of an *in vivo* Syphilis model, wherein the mean number of *T. pallidum* cells in aspirates of the lesions at the site of intradermal infection with *T. pallidum* Chicago strain was investigated using darkfield microscopy in three groups of infected rabbits (each group n=2-3 animals) covering a Lefamulin treated group (left column for each time point), a control group treated with Benzathine penicillin G (middle column for each time point) and an untreated group (right column for each time point).

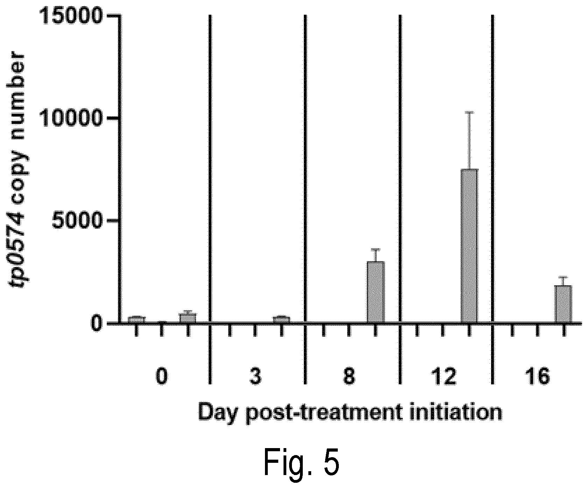

Figure 5 is a graph showing the effect of an exemplary compound of the present invention, Lefamulin, on the treponemal burden within the lesions of an *in vivo* Syphilis model, wherein the treponemal DNA (copy number of *T. pallidum* gene *tp0574)* in aspirates of the lesions at the site of intradermal infection with *T. pallidum* Chicago strain was investigated by quantitative PCR in three groups of infected rabbits (each group n=2-3 animals) covering a Lefamulin treated group (left column for each time point), a control group treated with Benzathine penicillin G (middle column for each time point) and an untreated group (right column for each time point).

### DETAILED DESCRIPTION OF THE INVENTION

Lefamulin is the INN for a compound of generic formula (I), more particular, Lefamulin is a compound of formula (VII) 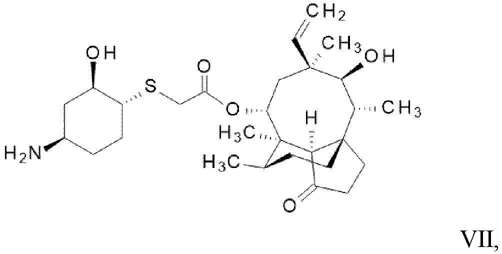 i.e. 14-O-{[(1*R,* 2*R*, 4*R*)-4-amino-2-hydroxy-cyclohexylsulfanyl]-acetyl}-mutilin (also known as "BC-3781").

In the following, the term "Lefamulin", when generally used without additional explanation, is intended to encompass both Lefamulin in free base form, as well as its salts and solvates.

Lefamulin has been developed for systemic use to treat serious bacterial infections in humans and was approved for medical use in the United States in 2019 to treat adults with community-acquired bacterial pneumonia (CABP).

It has been found that the compounds used according to the present invention have an antibacterial effect on several spirochetes or bacteria selected from the phylum of Spirochaetes. Bacteria of the phylum of Spirochaetes include pathogens responsible for infectious bacterial diseases in humans and animals.

As shown in the Examples 1-4, Lefamulin has an antimicrobial *in vitro* activity against bacteria of the phylum of Spirochaetes, which includes the bacteria known to mediate the bacterial infections. The antibiotic effect was particularly shown against bacteria strains of the genera *Borreliella* and *Treponema* (Examples 1 and 2). The investigated bacteria include bacteria which are known to cause bacterial infectious diseases in humans, in particular Lyme disease and Syphilis.

The data also demonstrate an antibacterial activity in an *in vivo* Rabbit model for syphilis (Examples 3 and 4).

Accordingly, the present invention concerns the compounds as defined in claims 1 to 6, especially Lefamulin, for use in the treatment or prevention of a bacterial infection mediated by spirochetes, more precisely bacteria selected from the phylum of Spirochaetes.

The phylum of Spirochaetes includes different taxonomic classes and orders. At the level of orders it includes i.a. Brachyspirales, Spirochaetales or Leptospirales. Brachyspirales include spirochetes known to cause veterinary disease including for example *Brachyspira hyodysenteriae.* Spirochaetales or Leptospirales also include bacteria mediating bacterial infections in humans. Within the order of Spirochaetales, there are the taxonomic families of Borreliaceae and Treponemataceae (among other families).

In a preferred embodiment of the present invention, the present invention concerns the compound as defined in claims 1 to 6, especially Lefamulin, for use in the treatment or prevention of a bacterial infection mediated by bacteria selected from the orders of Spirochaetales or Leptospirales, more preferably Spirochaetales. In a particular embodiment, the bacteria are selected from the families of Borreliaceae and Treponemataceae (both within the order of Spirochaetales).

More preferably, the bacteria are selected from the group consisting of the genera *Borrelia,*

*Borreliella, Leptospira,* and *Treponema* preferably from the group consisting of the genera *Borreliella* and *Treponema.*

If referred to a specific genus, it is to be understood that the term includes all species and subspecies of the genus. For example, the genus *Borrelia* covers *Borrelia spp.*

Bacteria of the genus *Borrelia* within the order of Spirochaetales and the family of Borreliaceae cause Relapsing fever. The disease is characterized by relapsing fevers with spirochetes evident on blood smear and transmitted e.g. by bites of lice or soft-bodied ticks (genus Ornithodoros). Particular bacteria of interest include: *Borrelia crocidurae, Borrelia duttoni, Borrelia hermsii, Borrelia ispanica, Borrelia miyamotoi, Borrelia parkeri, Borrelia turicatae, Borrelia persica,* and *Borrelia recurrentis.*

Bacteria of the genus *Borreliella* were formerly referred to as Borrelia, however, represent an individual genus within the order of Spirochaetales and the family of Borreliaceae. Bacteria of the genus *Borreliella* cause Lyme Disease / Lyme Borreliosis. Lyme borreliosis (LB) is a tick-transmitted bacterial infection caused by some members of the spirochete group *Borreliella burgdorferi.* It is the most prevalent tick-transmitted infection in temperate areas of Europe, North America and Asia, and its geographic distribution is ever-increasing. The B. *burgdorferi* complex comprises at least 15 genospecies worldwide; still, only six are significantly pathogenic to humans. All pathogenic genospecies can cause erythema migrans, the early skin rash of LB. *B. afzelii* and *B. garinii* are the major pathogenic genospecies found in Europe and are associated with skin and neurological complications, respectively. B. *burgdorferi* sensu stricto (the major pathogenic genospecies found in North America) is present in some parts of Europe and can cause neurological and arthritic complications. In North America, *Borreliella mayonii* (also referred to as *Borrelia mayonii)* are a type of bacteria recently (2013) found that can cause Lyme disease. Based on limited information, illness caused by *B. mayonii* appears similar to that caused by *B. burgdorferi,* but with a few differences. Like *B. burgdorferi, B. mayonii* causes fever, headache, rash, and neck pain in the days after infection and can cause arthritis after a few weeks of illness. Unlike *B. burgdorferi, B. mayonii* can also cause nausea and vomiting; large, widespread rashes; and a higher concentration of bacteria in the blood. Other pathogenic genospecies have been identified in Europe: *B. bavariensis,* associated with neurological complications, and *B. spielmanii.* In a preferred embodiment, the bacteria are *Borreliella* selected from the group of the species mentioned in this paragraph, and more preferably *Borreliella burgdorferi* and *Borreliella garinii.*

In a preferred embodiment, the bacterial infection is mediated by bacteria of the family of Borreliaceae, preferably by *Borreliella* or *Borrelia,* more preferably *Borreliella.* In particular, the bacterial infection is selected from the group of Lyme Disease and Relapsing fever, preferably Lyme Disease (including Lyme Borreliosis).

Bacteria of the genus *Leptospira* within the order of Leptospirales and the family of Leptospiraceae cause Leptospirosis. Leptospirosis is a bacterial disease/infection that affects humans and animals. A rare and severe form of human Leptospirosis includes Weil's disease with symptomes like chest pain and swollen arms and legs. It often requires hospitalization.

Currently, the genus *Leptospira* includes 21 named species of e.g. *Leptospira interrogans, Leptospira inadai.*

Bacteria of the genus *Treponema* within the order of Spirochaetales and the family of Treponemataceae cause various diseases in humans also referred to as treponematoses.

Syphilis is a complex systemic illness caused by the highly invasive *Treponema pallidum. Treponema pallidum* subsp *pallidum* mediates venereal syphilis (the classical form of sexually transmitted syphilis); *T. pallidum* subsp *endemicum* mediates endemic syphilis. *T pallidum* subsp *pertenue* mediates yaws. Yaws is a common chronic infectious disease that occurs mainly in warm humid regions. The disease has many names (for example, pian, parangi, paru, frambesia tropica). Yaws usually features lesions that appear as bumps on the skin of the face, hands, feet, and genital area.

*Treponema carateum* mediates the infectious disease Pinta, a skin infection, which occurs only in the Western hemisphere, has been described in Central and South America, Cuba, and the Caribbean islands. Pinta is the most benign of the nonvenereal treponematoses, because it involves only the skin.

*Treponema denticola* is associated with the incidence and severity of human periodontal disease (treponemal peridontitis). Having elevated *T. denticola* levels in the mouth is considered one of the main etiological agents of periodontitis.

In a preferred embodiment, the bacteria are *Treponema* selected from the group of the species mentioned in the preceding paragraphs, and more preferably *Treponema pallidum.*

In a preferred embodiment, the bacterial infection is mediated by *Treponema.* In particular, the bacterial infection is selected from the group of syphilis including venereal and endemic syphilis, pinta, (treponemal) periodontitis and yaws, preferably syphilis.

A subject in need of a treatment as contemplated by the present invention may be any living subject suffering from a bacterial infection mediated by spirochetes or by bacteria selected from the phylum of Spirochaetes. Especially, the subject may be a human or an animal, in particular a human.

In one embodiment, the compound is administered (or configured for being administered) to a human.

Treating, treatment or to treat as understood herein includes on one hand the complete curing, curation or to cure a condition (the bacterial infection) such that it comes to its end and on the other hand also ameliorating, amelioration or to ameliorate a condition such that its symptoms are reduced at least partially or individually.

Treatment typically includes administering a compound as used according to the present invention to a subject in need thereof, e.g. in one embodiment, a subject being diagnosed to have a bacterial infection mediated by spirochetes or by bacteria selected from the phylum of Spirochaetes. The subject may have a medical history including earlier symptoms of the bacterial infection, i.e. resulting in a bacterial infection being diagnosed earlier and the current symptoms occurring later. Accordingly, in a preferred embodiment, the compound for use according to the present invention is administered to a subject showing symptoms of or being diagnosed with a bacterial infection mediated by spirochetes or by bacteria selected from the phylum of Spirochaetes.

Preventing, prevention, or to prevent includes administering a compound before a condition is diagnosed or before onset of (all) disease symptoms of the condition.

For example, prevention according to the present invention may be considered after a subject has been infected with spirochetes or bacteria selected from the phylum of Spirochaetes but has not shown any symptoms of a bacterial infection (asymptomatic carrier) or, wherein a subject has been exposed and/or is prone to exposition to the bacteria.

The appropriate dosage of the compound to be administered according to the present invention, in particular Lefamulin, will, of course, vary depending upon, for example, the individual host, the mode of administration and the nature and severity of the conditions being treated. However, in general, for satisfactory results in larger mammals, for example humans, an indicated daily dosage is in the range from about 0.5 mg to 3 g of a compound used according to the present invention conveniently administered, for example, in divided doses up to four times a day.

The compound used according to the present invention may be administered by any conventional route, for example enterally, e.g. including nasal, buccal, rectal, oral administration; parenterally, e.g. including intravenous, intramuscular, subcutaneous, transdermal administration; or topically, e.g. including pulmonary, epicutaneous, intranasal, intratracheal, dermal administration, e.g. in form of coated or uncoated tablets, capsules, injectable solutions or suspensions, e.g. in the form of ampoules, vials, in the form of ointments, creams, gels, pastes, inhaler powder, foams, tinctures, lip sticks, drops, sprays, patches, or in the form of suppositories. In particular, route and form of administration may be selected in analogous manner to antibiotic agents such as e.g. tetracyclines, beta-lactam antibiotics including penicillins and cephalosporins, macrolides, and oxazolidinones.

Preferably, the compound used according to the present invention is administered intravenously (IV) or orally.

Preferred pharmaceutical compositions of Lefamulin for injection are disclosed in WO 2016/202788 A1.

The compound used according to the present invention, in particular Lefamulin, may be administered in the form of a pharmaceutically acceptable salt, e.g. an acid addition salt, or in free form, optionally in the form of a solvate.

In one embodiment, the compound is in the form of a salt and/or a solvate.

A salt of a compound used according to the present invention includes an acid addition salt.

Pharmaceutically acceptable acid addition salts include salts of a compound used according to the present invention with an acid, e.g. hydrogen fumaric acid, fumaric acid, tartaric acid, ethane-1,2-disulphonic acid, maleic acid, naphthalin-1,5-sulphonic acid, acetic acid, malic acid, lactic acid, i.e., L-lactic acid, succinic acid, salicylic acid, azelaic acid, 2-[(2,6-dichlorophenyl)amino]benzene acetic acid, hydrochloric acid, deuterochloric acid, and citric acid, preferably hydrochloric acid, acetic acid, L-lactic acid and maleic acid. In a further embodiment, the compound of the invention may be used as acid addition salt with itaconic acid, e.g. Lefamulin itaconate salt (WO 2021/209174).

In a preferred embodiment, the compound used according to the invention is Lefamulin in the form as Lefamulin acetate salt.

Preferred crystalline forms of Lefamulin as well as crystalline salt forms of Lefamulin are disclosed in WO 2011/146954 A1.

Of these, the acetate salt of Lefamulin in crystalline Form B as disclosed in WO 2011/146954 A1 is especially preferred.

The compound used according to the present invention, in particular Lefamulin, may be used for the pharmaceutical treatment contemplated herein alone or in combination with one or more other pharmaceutically active agents. Such other pharmaceutically active agents include those used in the standard-of-care therapy of infections caused by spirochetes, e.g. other antibacterial compounds such as e.g. tetracyclines, beta-lactam antibiotics including penicillins and cephalosporins, macrolides, oxazolidinones.

Combinations include fixed combinations, in which two or more pharmaceutically active agents are in the same formulation; kits, in which two or more pharmaceutically active agents in separate formulations are sold in the same package, e.g. with instruction for coadministration; and free combinations in which the pharmaceutically active agents are packaged separately, but instruction for simultaneous or sequential administration are given.

A pharmaceutical composition comprising a compound used according to the present invention, in particular Lefamulin may in addition comprise at least one pharmaceutically acceptable excipient, e.g. carrier or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers.

Such pharmaceutical compositions may be manufactured according, e.g. analogously, to a method as conventional, e.g. by mixing, granulating, coating, dissolving, spray drying, or lyophilizing processes. Unit dosage form may contain, for example, from about 0.5 mg to about 3000 mg, such as 10 mg to about 600 mg.

### Examples

Herein, including the examples, the following abbreviations are used:
- ATCC: American Type Culture Collection
- BPG: Benzathine penicillin G
- BSK: Barbour-Stonner-Kelly
- CLSI: Clinical and Laboratory Standards Institute
- FTA-ABS: Fluorescent Treponemal Antibody-Absorption test (Trinity Biotech, Jamestown, NY, USA).
- HPRT: hypoxanthine guanine phosphoribosyltransferase
- ID: intradermal
- IM: intramuscular
- MIC: Minimal Inhibitory Concentration [µg/mL]
- PCR: Polymerase Chain Reaction
- qPCR: quantitative Polymerase Chain Reaction
- RIT: rabbit infectivity test
- SD: standard deviation
- TPPA: *Treponema pallidum* Particle Agglutination test (Fujirebio, Malvern, PA, USA)
- VDRL: Venereal Disease Research Laboratory test (Becton-Dickinson, Franklin Lanes, NJ, USA)

### Example 1 - In vitro Activity against Borreliella burgdorferi and Borreliella garinii

**Objective:** To evaluate the antibacterial *in vitro* activity of Lefamulin and antibiotic comparators against several *Borreliella* strains.

### Methodology:

Minimal inhibitory concentrations (MIC) were determined against five (n=5) *Borreliella burgdorferi* isolates (ATCC 51990, 55131, 35211, 35210, 53899) and one (n=1) *Borreliella garinii* isolate (ATCC 51991) by broth microdilution technique (Dever, L.L., Jorgensen, J.H., Barbour, A.G. In vitro antimicrobial susceptibility testing of Boreliella burgdorferi: a microdilution MIC method and time-kill studies. J Clin Microbiol. 30(10), 2692-2697. (1992) doi: 10.1128/jcm.30.10.2692-2697.1992; Feng, J., Wang, T., Shi, W., et al. Identification of novel activity against Boreliella burgdorferi persisters using an FDA approved drug library. Emerg Microb Infect 3, e49 (2014) doi: 10.1038/emi.2014.53). The general procedures for broth microdilution were done according to the CLSI guidelines M7 and M100-S31 (CLSI. (2018) Methods for dilution antimicrobial susceptibility tests for bacteria that grow aerobically; Eleventh edition. M07.; CLSI. (2021) Performance Standards for Antimicrobial Susceptibility Testing; Thirty-first Edition. M100-S31).

Briefly, *Borreliella* spp. were grown at 36°C under microaerophilic conditions in BSK II medium until the logarithmic or stationary growth phase was reached. The growth phase was determined by semiquantitative measurement of active and dormant cells by fluorescence microscopy of cells stained with the fluorescent dyes SYBR Green I and propidium iodide. An inoculum of 10⁵-10⁶ spirochetes/mL was used to inoculate broth microdilution plates containing serially 2-fold diluted test compounds. The inoculated microdilution plates were then covered with an adhesive film to ensure microaerophilic culture conditions and incubated at 36°C for 5 days or until bacterial growth was observed in the positive control wells. MICs for the logarithmic growth phase cultures were read after 5 or 6 days of incubation (when the growth control showed good growth) and MICs for the stationary growth phase cultures were read after 8 days of incubation.

### Results:

The resulting MIC values for the logarithmic and stationary growth phase cultures are summarized in Table 1 below. MIC results for single isolates varied slightly in various experiments but were within one 2-fold dilution factor (or at most two). Accordingly, the MIC values are indicated with ranges covering different experiments against *Borreliella* spp. (n=6) including *B. burgdorferi* (n=5) and *B. garinii* (n=1) in either logarithmic or stationary growth phase (Table 1).

Lefamulin was one of the most active compounds when compared to other antibiotic classes that are used to treat Lyme disease including doxycycline, ceftriaxone, cefuroxime or azithromycin. The lefamulin MICs ranged between ≤0.001-0.008 µg/mL for cultures in logarithmic growth phase; for cultures in stationary growth phase the MIC range was similar (≤0.001-0.008 µg/mL). The MIC values of Pleuromutilin were higher in about two orders of magnitude. Tiamulin showed better MIC values in comparison to Pleuromutilin, however, was clearly outperformed by Lefamulin.

**Table 1. In vitro antibacterial activity of Lefamulin and comparator antibiotics against Borreliella spp.**

| **Compound** | **MIC (µg/mL) for Logarithmic Growth Phase Culture** | **MIC (µg/mL) for Stationary Growth Phase Culture** |
|---|---|---|
| | (n=6) | (n=6) |
| Lefamulin | ≤0.001-0.008 | ≤0.001-0.008 |
| Doxycycline | ≤0.03-0.5 | ≤0.03-0.5 |
| Erythromycin | 0.002-0.016 | ≤0.001-0.004 |
| Azithromycin | ≤0.001-0.004 | ≤0.001-0.004 |
| Amoxicillin | ≤0.03-0.12 | ≤0.03-0.12 |
| Cefuroxime | 0.016-0.25 | 0.016-0.25 |
| Ceftriaxone | ≤0.004-0.016 | ≤0.004-0.03 |
| Ciprofloxacin | 0.25-2 | 0.25-2 |
| Levofloxacin | 2-4 | 2-8 |
| Pleuromutilin | 0.12-1 | 0.12-2 |
| Tiamulin | ≤0.03-0.5 | ≤0.03-0.25 |

### Example 2 - In vitro Susceptibility and Infectivity Test with Treponema pallidum

**Objective:** To determine the *in vitro* efficacy and MICs of Lefamulin against *T. pallidum* an *in vitro* propagation system based on the co-cultivation of treponemes and rabbit Sf1 epithelial (Sf1Ep) cells that can continually support *T. pallidum* growth outside of a susceptible host.

### Methodology:

The *in vitro* susceptibility determination essentially followed the protocol described by Edmondson *et al.* (Edmondson, D.G., Hu, B., Norris, S.J. Long-Term In Vitro Culture of the Syphilis Spirochete Treponema pallidum subsp. pallidum. mBio 9(3) e01153-18. (2018). doi: 10.1128/mBio.01153-18).

Briefly, 3x10³ rabbit Sf1Ep cells seeded in 96-well microtiter plates in 150 µl of culture media, were inoculated with the *T. pallidum* strains Chicago and SS14 at an inoculum of 3x10³ (counted using dark field microscopy) in TpCM-2 medium that was equilibrated overnight at 34°C in a microaerophilic environment consisting of 1.5% O₂, 3.5% CO₂ and 95% N₂ supplied as a tri-gas mix. Lefamulin and penicillin G were added from 100-fold concentrated stocks in water to achieve the final test concentrations of 5, 2.5, 0.5, 0.1, 0.02. 0.01. 0.005, and 0.0025 µg/ml for Lefamulin and 60 ng/ml for penicillin G. Then the culture plates were incubated at 34°C in a tri-gas incubator. Cells were then lysed and the DNA extracted using a Quick DNA-96 kit followed by the quantification of the treponemes by quantitative real-time PCR in triplicate targeting the *tp0574* gene (Giacani L, Molini B, Godornes C, Barrett L, Van Voorhis WC, Centurion-Lara A, et al. Quantitative analysis of tpr gene expression in Treponema pallidum isolates: differences among isolates and correlation with T-cell responsiveness in experimental syphilis. Infect Immun. 75(1), 104-12 (2007). doi: 10.1128/IAI.01124-06).

### Results:

The results in terms of mean copy number of the *tp0574* gene and its standard deviation (SD) for the experiments with *T. pallidum* strain Chicago and SS14 are shown in Figure 1 and 2, respectively.

Lefamulin was highly effective against the *T. pallidum* Chicago strain at a minimal inhibitory concentration (MIC) of ≥10 ng/ml showing mean copy numbers of 324-403 copies/µL (inoculum on day 0 was 539 copies/mL), whereas the untreated control reached a mean copy number of 13,052 copies/µL on day 7 (Figure 1). Similarly, the *T. pallidum* SS14 strain was inhibited at Lefamulin concentrations of 10-20 ng/mL or higher. At ≥10 ng/mL Lefamulin, mean copy numbers of 33-124 copies/µL (inoculum on day 0 was 170 copies/µL) were determined, whereas the untreated control reached a mean copy number of 894 copies/µL on day 7 (Figure 2). As expected, the positive control penicillin G was highly effective at 60 ng/mL for both strains, Chicago and SS14, displaying copy numbers of 247 copies/µL and 91 copies/µL, respectively (Figures 1 and 2).

### Example 3 - In vivo Activity in a Rabbit Model for Treponema pallidum infection

**Objective:** To evaluate the potential of Lefamulin in treatment of syphilis *in vivo* in a rabbit model by monitoring of the cutaneous lesions and treponemal burden at the site of infection.

### Methodology:

The preclinical study to test Lefamulin against *T. pallidum* was conducted in analogy to an investigation of the antibiotic compound linezolid (Haynes, A.M., Giacani, L., Vall Mayans, M., et al. Efficacy of linezolid on Treponema pallidum, the syphilis agent: A preclinical study. eBioMedicine 65, 103281 (2021). doi: 10.1016/j.ebiom.2021.103281).

In the syphilis infection model, each animal was infected intradermally (ID) with 10⁶ freshly harvested, viable and infectious *T. pallidum* cells (Chicago strain) per site of infection on 10 sites on their shaved backs to induce appearance of primary syphilis lesions. Treponemal viability prior to ID infection was assessed by dark field microscopy (DFM). A dose of infectious treponemes (10⁶/site of infection) induces the appearance of an erythematous lesion at the inoculation site within ~5 days post-inoculation. Overall, the arch of lesion progression from the erythematous stage to the indurated, ulcerated, and healed stages encompasses approximately 4 weeks.

Herein, three groups of three rabbits were infected. Three animals were treated orally with 70 mg/kg Lefamulin every 12 h for 3 days (day 7 to day 10 post-infection). As positive control group, another three animals were treated with Benzathine penicillin G (BPG) as single intramuscular (IM) injection (300,000 units; equivalent w/w to 4.8 million units for humans). One group (n=3) served as untreated control. The cutaneous lesions were monitored macroscopically for clinical development, documented by photography on regular basis and measured for their diameter every day for a period of 40 days.

Treponemal burden within these lesions was assessed by taking needle aspirates from the lesions and analyzing the aspirates for pathogen cell count using DFM as well as quantification of treponemal DNA extracted from the aspirates at day 3, 8, 12, and 16 Additionally, mRNA extracted from lesion biopsies obtained post-treatment initiation was also quantified as a surrogate of viability. The gene copy number of the *T. pallidum tp0574* gene was quantified by qPCR. Additionally, the tp0574 gene message was normalized to the message of the rabbit housekeeping gene HPRT to evaluate the burden of viable *T. pallidum* cells within lesion biopsies.

### Results:

The results from monitoring the indurated/ulcerated lesions in treated and control rabbit post-ID infection challenge are shown in Figure 3 and summarized herein below. In the Lefamulin-treated group one rabbit died on day 3 of treatment, most likely due to inhalation of Lefamulin caused by reflux. Thus, the results are limited to the two remaining animals.

Lesion diameter in Lefamulin-treated animals was found to be significantly lower (p<0.05) compared to the untreated controls already at day 10 post-infection, at the end of the treatment window.

In animals treated with BPG and Lefamulin, lesions quickly reverted from slightly indurated to flat and hence no longer measurable. In Lefamulin-treated animals, only 14% of the lesions were still measurable by day 9 post-treatment initiation, and no induration could be measured at any site by day 13 post-treatment initiation. The resolution of the lesions in the BPG-treated group was slightly faster (95% of the measured sites resolved at day 9 post-BPG administration and 100% of the measured sites by day 12 post-treatment). None of the lesions in these two groups progressed past the initial state of mild induration. Macroscopically, there were no differences between BPG- and Lefamulin- treated animals at this timepoint.

On the contrary, all lesions from untreated controls ulcerated by day 19 post-inoculation. Ulceration was seen starting at day 17 post-inoculation in all animals. The rabbits were observed for a period of 40 days in total. However, Figure 3 is limited to the first 30 days post-inoculation (i.e., post-challenge) as there were no further changes in the treatment groups and the mean diameter of the lesion in the control groups remained at about 11 to 14 mm until day 40 post-challenge.

The treponemal burden in extracts from the lesions was investigated at days 0, 3, 8, 12 and 16 after initiation of the treatment and the results are shown in Figures 4 and 5 and summarized herein below.

The mean number of *T. pallidum* cells determined by DFM showed that no treponemal bacteria could be found in aspirates from Lefamulin- and BPG-treated animals since day 3 post-treatment initiation (left and middle column for each day in Figure 4, respectively). As expected, in control animals, treponemal burden increased until day 19 post-infection (i.e., day 12 post-treatment initiation) to then decline due to naturally occurring immune clearance of the pathogen (right column for each day in Figure 4). The same trends were obtained when aspirate suspensions were analyzed using qPCR targeting the tp0574 gene of *T. pallidum* (Figure 5).

The effect of Lefamulin and BPG treatment was also confirmed in the analysis of the tp0574 gene message (normalized to the message of the rabbit housekeeping gene HPRT) to evaluate the burden of viable *T. pallidum* cells within lesion biopsies. *T. pallidum* mRNA was absent in all lesions obtained from BPG- and lefamulin-treated rabbits already at day 3 post-treatment initiation (data not shown).

In summary, the results confirmed the efficacy of Lefamulin against *T. pallidum* in an infection model in rabbits following three days of treatment with 70 mg/kg orally twice daily.

### Example 4 - Rabbit infectivity test

**Objective:** To evaluate the ability of Lefamulin treatment in prevention of treponemal dissemination to lymph nodes through a rabbit infectivity test (RIT) with this tissue.

### Methodology:

The test rabbits from Example 3 were used for a subsequent study in a RIT, wherein samples from the popliteal lymph nodes were transferred to new (naive) rabbits, which were subsequently analyzed for disease symptoms and tested serologically (Haynes, A.M., Giacani, L., Vall Mayans, M., et al. Efficacy of linezolid on Treponema pallidum, the syphilis agent: A preclinical study. eBioMedicine 65, 103281 (2021). Doi: 10.1016/j.ebiom.2021.103281).

Following euthanasia, popliteal lymph nodes (LN) from all the rabbits in the Lefamulin, BPG, and control groups were harvested. Lymph nodes from each rabbit were pooled and homogenized in a solution containing equal amounts of sterile saline and serum from an uninfected rabbit. One milliliter of homogenate from each test rabbit was then inoculated into the left testicle of a naive rabbit to perform the RIT. For the rabbit, which died at day 3 post-treatment initiation, lymph nodes were harvested and transferred to a RIT rabbit within 1 hour after the animal's death.

Inoculated animals were checked for orchitis twice weekly. Serum was collected monthly for three months and analyzed with two different serological tests, i.e. VDRL (Venereal Disease Research Laboratory; Becton-Dickinson, Franklin Lanes, NJ) and TPPA *(Treponema pallidum* Particle Agglutination; Fujirebio, Malvern, PA). Also an FTA-ABS test (Fluorescent Treponemal Antibody-Absorption; Trinity Biotech, Jamestown, NY) was performed.

RIT rabbits were euthanized either after developing orchitis or becoming VDRL positive, or at the end of the three-month observation period if they did not show serological or clinical signs of infection.

### Results:

Shortly, the RIT rabbits challenged with minced popliteal lymph nodes from the test rabbits from the group treated with either Lefamulin or BPG did not show any signs of disease. None of the animals developed orchitis or seroconverted throughout the 3-months observation period.

The animals that received lymph nodes harvested from the untreated control rabbits became infected approximately a month following inoculation. Two animals developed orchitis at day 23 and 30 post-inoculation, and treponemes were visible under DFM when lesion aspirated were analyzed. A third animal became VDRL and TPPA positive (titers of 1:4 and 1:2560, respectively) at day 30 post-inoculation, but did not develop orchitis. In rabbits with orchitis, treponemes were also detected in testicular aspirates using DFM. Orchitic checks were performed throughout the duration of the 3-month observation period, until the animals developed orchitis or seroconverted.

To further confirm lack of treponemal antibodies, in addition to TPPA, also FTA-ABS a test was performed. All control animals showed a 3+ reactivity (or higher), while none of the BGP or Lefamulin treated animals seroconverted.

In summary, Lefamulin treatment prevented treponemal dissemination to lymph nodes as effective as treatment with BPG.

## Claims

1. A compound of formula (I) 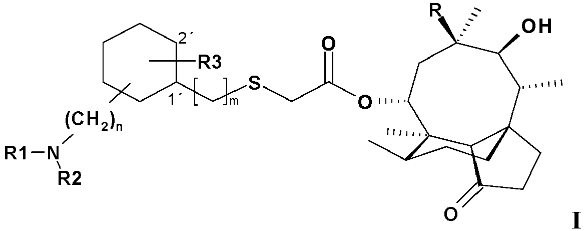 wherein
n is 0 to 4;
m is 0 or 1 with the proviso that the sulphur atom and R₃ are in vicinal position (if m = 0 then R₃ is in position 2', and if m = 1 then R₃ is on position 1 ');
R is ethyl or vinyl;
R₁ is hydrogen or (C₁₋₆)alkyl,
R₂ is hydrogen or
- (C₃₋₆)cycloalkyl, or
- unsubstituted (C₁₋₆)alkyl, or
- (C₁₋₆)alkyl substituted by one or more of
- hydroxy; preferably one or two,
- methoxy,
- halogen, and
- (C₃₋₆)cycloalkyl, or
R₁ and R₂ together with the nitrogen atom to which they are attached form a 5 to 7 membered heterocyclic ring containing at least 1 nitrogen atom or 1 nitrogen and 1 additional heteroatom e. g. selected from N or O, or
R₁ is hydroxy and R₂ is formyl;
R₃ is OH, OR₄, a halogen atom, or
R₃ is bound to 2' and represents -O-(CH₂)ₚ-O- with p is 2 or 3;
R₄ is unsubstituted (C₁₋₆)alkyl or (C₃₋₆)cycloalkyl,
or a pharmaceutically acceptable salt or solvate thereof
for the specific use in the treatment or prevention of a bacterial infection mediated by spirochetes.

2. Compound for use according to claim 1, wherein the compound is selected from the group consisting of the compounds of formula (II), (III), (IV), (V) and (VI) 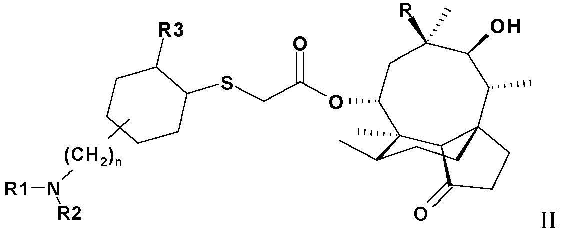 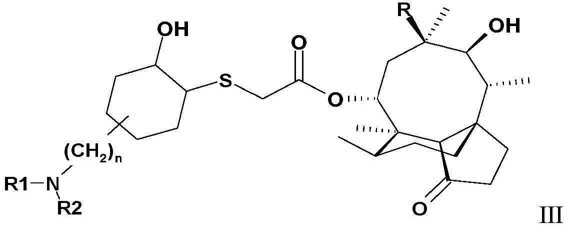 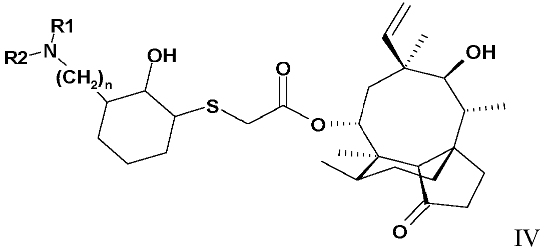 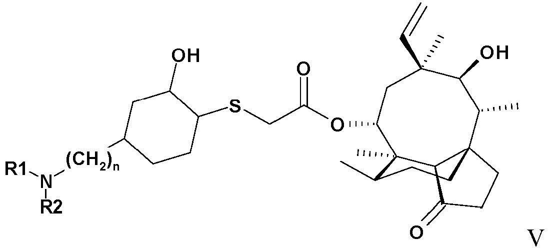 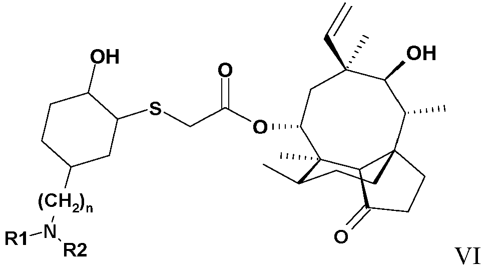 wherein in each case n, R₁ and R₂ are as defined in claim 1.

3. A compound for use according to claim 1 or 2, wherein the compound is selected from the group consisting of
14-O-{[(1R, 2R, 4R)-4-Amino-2-hydroxy-cyclohexylsulfanyl]-acetyl}-mutilin
14-O-{[(1S, 2S, 4S)-4-Amino-2-hydroxy-cyclohexylsulfanyl]-acetyl}-mutilin
14-O-{[(1R, 2R, 5S)-5-Amino-2-hydroxy-cyclohexylsulfanyl]-acetyl}-mutilin
14-O-{[(1S, 2S, 5R)-5-Amino-2-hydroxy-cyclohexylsulfanyl]-acetyl}-mutilin
14-O-{[(1R, 2R, 4S)-4-Amino-2-hydroxy-cyclohexylsulfanyl]-acetyl}-mutilin and the (1S, 2S, 4R) diastereomer thereof
14-O-{[(1R, 2R, 5R)-5-Amino-2-hydroxy-cyclohexylsulfanyl]-acetyl}-mutilin
14-O-{[(1S, 2S, 5S)-5-Amino-2-hydroxy-cyclohexylsulfanyl]-acetyl}-mutilin
14-O-{[(1R, 2R, 3R)-3-Amino-2-hydroxy-cyclohexylsulfanyl]-acetyl}-mutilin and the (1S, 2S, 3S) diastereomer thereof
14-O-{[(1R, 2R, 4R)-4-Diethylamino-2-hydroxy-cyclohexylsulfanyl]-acetyl}-mutilin and the (1S, 2S, 4S) diastereomer thereof
14-O- {[(1R, 2R, 4R)-4-Ethylamino-2-hydroxy-cyclohexylsulfanyl]-acetyl}-mutilin and the (1S, 2S, 4S) diastereomer thereof
14-O-{[(1R, 2R, 5S)-5-Ethylamino-2-hydroxy-cyclohexylsulfanyl]-acetyl}-mutilin and the (1S, 2S, 5R) diastereomer thereof
14-O-{[(1R, 2R, 5S)-5-Diethylamino-2-hydroxy-cyclohexylsulfanyl]-acetyl}-mutilin and the (1S, 2S, 5R) diastereomer thereof
14-O-{[(1R, 2R, 4S)-4-Diethylamino-2-hydroxy-cyclohexylsulfanyl]-acetyl}-mutilin and the (1S, 2S, 4R) diastereomer thereof
14-O-{[(1R, 2R, 5R)-5-Diethylamino-2-hydroxy-cyclohexylsulfanyl]-acetyl}-mutilin and the (1S, 2S, 5S) diastereomer thereof
14-O-{[(1R, 2R, 3R)-3-Ethylamino-2-hydroxy-cyclohexylsulfanyl]-acetyl}-mutilin and the (1S, 2S, 3S) diastereomer thereof
14-O-{[(1R, 2R, 3R)-3-Diethylamino-2-hydroxy-cyclohexylsulfanyl]-acetyl}-mutilin and the (1S, 2S, 3S) diastereomer thereof
14-O-{[(1R, 2R, 4S)-4-(Formyl-hydroxy-amino)-2-hydroxy-cyclohexylsulfanyl]-acetyl }-mutilin and the (1S, 2S, 4R) diastereomer thereof
14-O-{[(1R, 2R, 5S)-5-(Formyl-hydroxy-amino)-2-hydroxy-cyclohexylsulfanyl]-acetyl }-mutilin and the (1S, 2S, 5R) diastereomer thereof
14-O-{[(1R, 2R, 3R/S)-3-(Formyl-hydroxy-amino)-2-hydroxy-cyclohexylsulfanyl]-acetyl }-mutilin and the (1S, 2S, 3R/S) diastereomer thereof
14-O-{[(1R, 2R, 5S)- 2-Hydroxy-5-methylamino-cyclohexylsulfanyl]-acetyl}-mutilin and the (1S, 2S, 5R) diastereomer thereof
14-O-{[(1R, 2R, 5S)-5-Allylamino-2-hydroxy-cyclohexylsulfanyl]-acetyl}-mutilin and the (1S, 2S, 5R) diastereomer thereof
14-O-{[(1R, 2R, 5S)-2-Hydroxy-5-(2-methoxy-ethylamino)-cyclohexylsulfanyl]-acetyl }-mutilin and the (1S, 2S, 5R) diastereomer thereof
14-O-1[(1R, 2R, 4R*)-2-Hydroxy-4-(2-hydroxy-ethylamino)-cyclohexylsulfanyl]-acetyl }-mutilin and the (1S, 2S, 4S*) diastereomer thereof
14-O-{[(1R, 2R, 4R*)-4-Cyclohexylamino-2-hydroxy-cyclohexylsulfanyl]-acetyl}-mutilin and the (1S, 2S, 4S*) diastereomer thereof
14-O-{[(1R, 2R, 4R*)-4-Cyclopropylamino-2-hydroxy-cyclohexylsulfanyl]-acetyl}-mutilin and the (1S, 2S, 4S*) diastereomer thereof
14-O-{[(1R, 2R, 5S*)-4-Cyclopropylamino-2-hydroxy-cyclohexylsulfanyl]-acetyl}-mutilin and the (1S, 2S, 5R*) diastereomer thereof
14-O-{[(1R, 2R, 4S*)-4-Cyclopropylamino-2-hydroxy-cyclohexylsulfanyl]-acetyl}-mutilin and the (1S, 2S, 4R*) diastereomer thereof
14-O-{[(1R, 2R, 5R*)-2-Hydroxy-5-morpholin-4-yl-cyclohexylsulfanyl]-acetyl}-mutilin and the (1S, 2S, SS*) diastereomer thereof
14-O-{[(1R, 2R, 5S*)-2-Hydroxy-5-morpholin-4-yl-cyclohexylsulfanyl]-acetyl}-mutilin and the (1S, 2S, 5R*) diastereomer thereof
14-O-{[(1R, 2R, 5S)-5-Amino-2-hydroxy-cyclohexylsulfanyl]-acetyl}-19,20-dihydro-mutilin and the (1S, 2S, 5R) diastereomer thereof
14-O-{[(1R, 2R, 5S)-5-Ethylamino-2-hydroxy-cyclohexylsulfanyl]-acetyl}-19,20-dihydro-mutilin and the (1S, 2S, 5R) diastereomer thereof
14-O-{[(1R, 2R, 5R)-5-Amino-2-hydroxy-cyclohexylsulfanyl]-acetyl}-19,20-dihydro-mutilin and the (1S, 2S, 5S) diastereomer thereof
14-O-{[(1R, 2R)-4-Aminomethyl-2-hydroxy-cyclohexylsulfanyl]-acetyl}-mutilin and the (1S, 2S) diastereomers thereof
14-O-{[5-Amino-2-chloro-cyclohexylsulfanyl]-acetyl}-mutilin
14-O-{[4-Amino-2-chloro-cyclohexylsulfanyl]-acetyl}-mutilin
14-O-[(4-Amino-1-hydroxy-cyclohexylmethylsulfanyl)-acetyl]-mutilin
14-O-{[(1R, 2R)-2-Hydroxy-5-(3-methylamino-propyl)-cyclohexylsulfanyl]-acetyl}-mutilin and the (1S, 2S) diastereomer thereof
14-O-{[(1R, 2R)-2-Hydroxy-4-(3-methylamino-propyl)-cyclohexylsulfanyl]-acetyl}-mutilin and the (1S, 2S) diastereomer thereof
14-O-{[(1R, 2R)-5-(3-Amino-propyl)-2-hydroxy-cyclohexylsulfanyl]-acetyl}-mutilin and the (1S, 2S) diastereomer thereof
14-O-{[(1R, 2R)-4-(3-Amino-propyl)-2-hydroxy-cyclohexylsulfanyl]-acetyl}-mutilin and the (1S, 2S) diastereomer thereof
14-O-{[(6R, 8R)-8-Amino-1,4-dioxa-spiro[4.5]dec-6-ylsulfanyl]-acetyl}-mutilin and the (6S, 8S) diastereomer thereof
14-O-{[4-Amino-2-methoxy-cyclohexylsulfanyl]-acetyl}-mutilin
and
14-O-{[5-Amino-2-methoxy-cyclohexylsulfanyl]-acetyl}-mutilin.

4. A compound for use according to any of claims 1 to 3, wherein the compound is Lefamulin.

5. A compound for use according to any of claims 1 to 4, wherein the compound is in the form of a salt and/or a solvate.

6. A compound for use according to any of claims 1 to 5, wherein the compound is Lefamulin in the form as Lefamulin acetate salt.

7. A compound for use according to any of the preceding claims, wherein the bacterial infection is mediated by bacteria selected from the phylum of Spirochaetes.

8. A compound for use according to claim 7, wherein the bacteria are selected from the order of Spirochaetales or Leptospirales.

9. A compound for use according to claim 7 or 8, wherein the bacteria are selected from the order of Spirochaetales.

10. A compound for use according to any of the claims 1 to 8, wherein the bacteria are selected from the group consisting of the genera *Borrelia, Borreliella, Leptospira,* and *Treponema* preferably from the group consisting of the genera *Borreliella* and *Treponema.*

11. A compound for use according to any of the claims 1 to 10, wherein the bacterial infection is mediated by *Treponema.*

12. A compound for use according to claim 11, wherein the bacterial infection is selected from the group of syphilis including venereal and endemic syphilis, pinta, (treponemal) periodontitis and yaws, preferably syphilis.

13. A compound for use according to any of the claims 1 to 9, wherein the bacterial infection is mediated by bacteria of the family of Borreliaceae, preferably by *Borreliella* or *Borrelia,* more preferably *Borreliella.*

14. A compound for use according to claim 13, wherein the bacterial infection is selected from the group of Lyme Disease and relapsing fever, preferably Lyme Disease (including Lyme Borreliosis).

15. A compound for use according to any of the preceding claims, wherein the compound is administered to a human.

## Patentansprüche

1. Verbindung der Formel (I) 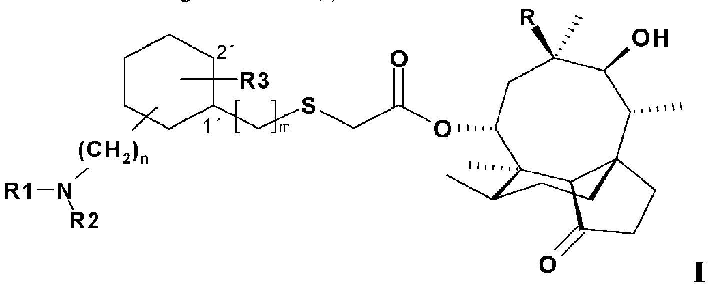 wobei
n 0 bis 4 ist;
m 0 oder 1 ist, unter der Voraussetzung, dass sich das Schwefelatom und R₃ in einer benachbarten Position befinden (wenn m = 0, dann befindet sich R₃ in Position 2', und wenn m = 1, dann befindet sich R₃ an Position 1');
R Ethyl oder Vinyl ist;
R₁ Wasserstoff oder (C₁₋₆)Alkyl ist;
R₂ Wasserstoff oder
- (C₃₋₆)Cycloalkyl oder
- unsubstituiertes (C₁₋₆)Alkyl oder
- (C₁₋₆)Alkyl, substituiert durch eines oder mehr von:
- Hydroxy; vorzugsweise eines oder zwei,
- Methoxy,
- Halogen und
- (C₃₋₆)Cycloalkyl, ist, oder
R₁ und R₂ zusammen mit dem Stickstoffatom, an das sie gebunden sind, einen 5- bis 7-gliedrigen heterocyclischen Ring bilden, der mindestens 1 Stickstoffatom oder 1 Stickstoff- und 1 zusätzliches Heteroatom, z.B. ausgewählt aus N oder O, enthält, oder
R₁ Hydroxy ist und R₂ Formyl ist;
R₃ OH, OR₄, ein Halogenatom ist, oder
R₃ an 2' gebunden ist und für -O-(CH₂)ₚ-O- steht, wobei p 2 oder 3 ist;
R₄ unsubstituiertes (C₁₋₆)Alkyl oder (C₃₋₆)Cycloalkyl ist,
oder ein pharmazeutisch verträgliches Salz oder Solvat davon,
zur spezifischen Verwendung bei der Behandlung oder Vorbeugung einer durch Spirochäten vermittelten bakteriellen Infektion.

2. Verbindung zur Verwendung gemäß Anspruch 1, wobei die Verbindung ausgewählt ist aus der Gruppe, bestehend aus den Verbindungen der Formel (II), (III), (IV), (V) und (VI) 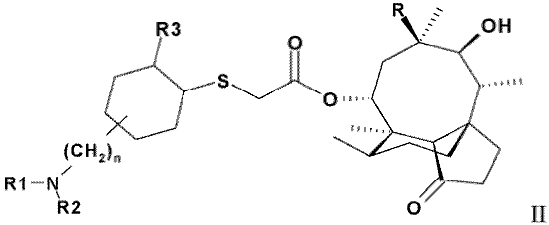 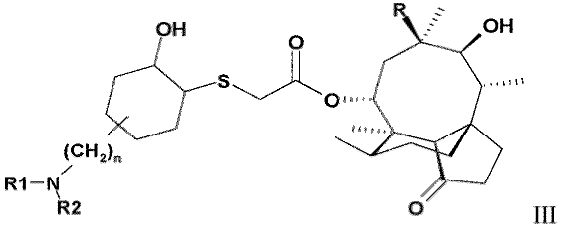 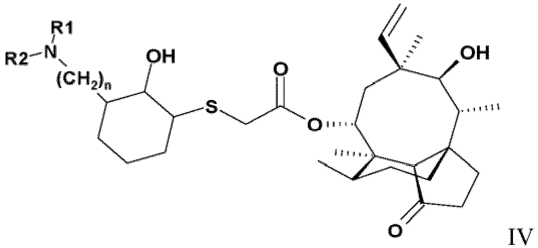 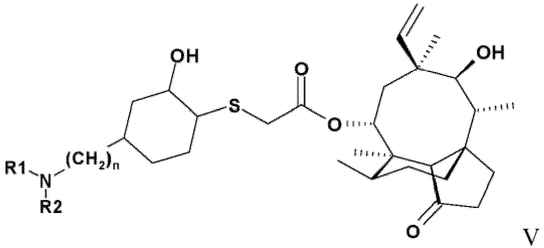 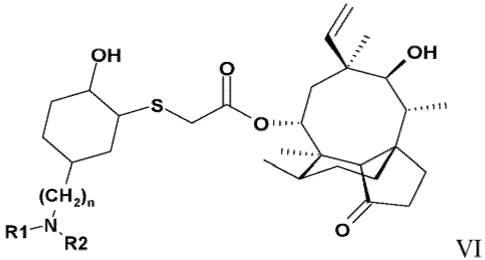 wobei jeweils n, R₁ und R₂ wie in Anspruch 1 definiert sind.

3. Verbindung zur Verwendung gemäß Anspruch 1 oder 2, wobei die Verbindung ausgewählt ist aus der Gruppe, bestehend aus
14-O-{[(1R,2R,4R)-4-Amino-2-hydroxycyclohexylsulfanyl]-acetyl}-mutilin
14-O-{[(1S,2S,4S)-4-Amino-2-hydroxycyclohexylsulfanyl]-acetyl}-mutilin
14-O-{[(1R,2R,5S)-5-Amino-2-hydroxycyclohexylsulfanyl]-acetyl}-mutilin
14-O-{[(1S,2S,5R)-5-Amino-2-hydroxycyclohexylsulfanyl]-acetyl}-mutilin
14-O-{[(1R,2R,4S)-4-Amino-2-hydroxycyclohexylsulfanyl]-acetyl}-mutilin und dem (1S,2S,4R)-Diastereomer davon
14-O-{[(1R,2R,5R)-5-Amino-2-hydroxycyclohexylsulfanyl]-acetyl}-mutilin
14-O-{[(1S,2S,5S)-5-Amino-2-hydroxycyclohexylsulfanyl]-acetyl}-mutilin
14-O-{[(1R,2R,3R)-3-Amino-2-hydroxycyclohexylsulfanyl]-acetyl}-mutilin und dem (1S,2S,3S)-Diastereomer davon
14-O-{[(1R,2R,4R)-4-Diethylamino-2-hydroxycyclohexylsulfanyl]-acetyl}-mutilin und dem (1S,2S,4S)-Diastereomer davon
14-O-{[(1R,2R,4R)-4-Ethylamino-2-hydroxycyclohexylsulfanyl]-acetyl}-mutilin und dem (1S,2S,4S)-Diastereomer davon
14-O-{[(1R,2R,5S)-5-Ethylamino-2-hydroxycyclohexylsulfanyl]-acetyl}-mutilin und dem (1S,2S,5R)-Diastereomer davon
14-O-{[(1R,2R,5S)-5-Diethylamino-2-hydroxycyclohexylsulfanyl]-acetyl}-mutilin und dem (1S,2S,5R)-Diastereomer davon
14-O-{[(1R,2R,4S)-4-Diethylamino-2-hydroxycyclohexylsulfanyl]-acetyl}-mutilin und dem (1S,2S,4R)-Diastereomer davon
14-O-{[(1R,2R,5R)-5-Diethylamino-2-hydroxycyclohexylsulfanyl]-acetyl}-mutilin und dem (1S,2S,5S)-Diastereomer davon
14-O-{[(1R,2R,3R)-3-Ethylamino-2-hydroxycyclohexylsulfanyl]-acetyl}-mutilin und dem (1S,2S,3S)-Diastereomer davon
14-O-{[(1R,2R,3R)-3-Diethylamino-2-hydroxycyclohexylsulfanyl]-acetyl}-mutilin und dem (1S,2S,3S)-Diastereomer davon
14-O-{[(1R,2R,4S)-4-(Formylhydroxyamino)-2-hydroxycyclohexylsulfanyl]-acetyl}-mutilin und dem (1S,2S,4R)-Diastereomer davon
14-O-{[(1R,2R,5S)-5-(Formylhydroxyamino)-2-hydroxycyclohexylsulfanyl]-acetyl}-mutilin und dem (1S,2S,5R)-Diastereomer davon
14-O-{[(1R,2R,3R/S)-3-(Formylhydroxyamino)-2-hydroxycyclohexylsulfanyl]acetyl}-mutilin und dem (1S,2S,3R/S)-Diastereomer davon
14-O-{[(1R,2R,5S)-2-Hydroxy-5-methylaminocyclohexylsulfanyl]acetyl}-mutilin und dem (1S,2S,5R)-Diastereomer davon
14-O-{[(1R,2R,5S)-5-Allylamino-2-hydroxycyclohexylsulfanyl]acetyl}-mutilin und dem (1S,2S,5R)-Diastereomer davon
14-O-{[(1R,2R,5S)-2-Hydroxy-5-(2-methoxyethylamino)-cyclohexylsulfanyl]-acetyl}-mutilin und dem (1S,2S,5R)-Diastereomer davon
14-O-{[(1R,2R,4R*)-2-Hydroxy-4-(2-hydroxyethylamino)-cyclohexylsulfanyl]-acetyl}-mutilin und dem (1S,2S,4S*)-Diastereomer davon
14-O-{[(1R,2R,4R*)-4-Cyclohexylamino-2-hydroxycyclohexylsulfanyl]-acetyl}-mutilin und dem (1S,2S,4S*)-Diastereomer davon
14-O-{[(1R,2R,4R*)-4-Cyclopropylamino-2-hydroxycyclohexylsulfanyl]-acetyl}-mutilin und dem (1S,2S,4S*)-Diastereomer davon
14-O-{[(1R,2R,5S*)-4-Cyclopropylamino-2-hydroxycyclohexylsulfanyl]-acetyl}-mutilin und dem (1S,2S,5R*)-Diastereomer davon
14-O-{[(1R,2R,4S*)-4-Cyclopropylamino-2-hydroxycyclohexylsulfanyl]-acetyl}-mutilin und dem (1S,2S,4R*)-Diastereomer davon
14-O-{[(1R,2R,5R*)-2-Hydroxy-5-morpholin-4-yl-cyclohexylsulfanyl]-acetyl}-mutilin und dem (1S,2S,5S*)-Diastereomer davon
14-O-{[(1R,2R,5S*)-2-Hydroxy-5-morpholin-4-yl-cyclohexylsulfanyl]-acetyl}-mutilin und dem (1S,2S,5R*)-Diastereomer davon
14-O-{[(1R,2R,5S)-5-Amino-2-hydroxycyclohexylsulfanyl]-acetyl}-19,20-dihydro-mutilin und dem (1S,2S,5R)-Diastereomer davon
14-O-{[(1R,2R,5S)-5-Ethylamino-2-hydroxycyclohexylsulfanyl]-acetyl}-19,20-dihydro-mutilin und dem (1S,2S,5R)-Diastereomer davon
14-O-{[(1R,2R,5R)-5-Amino-2-hydroxycyclohexylsulfanyl]-acetyl}-19,20-dihydro-mutilin und dem (1S,2S,5S)-Diastereomer davon
14-O-{[(1R,2R)-4-Aminomethyl-2-hydroxycyclohexylsulfanyl]-acetyl}-mutilin und den (1S,2S)-Diastereomeren davon
14-O-{[5-Amino-2-chlorcyclohexylsulfanyl]-acetyl}-mutilin
14-O-{[4-Amino-2-chlorcyclohexylsulfanyl]-acetyl}-mutilin
14-O-[(4-Amino-1-hydroxycyclohexylmethylsulfanyl)-acetyl]-mutilin
14-O-{[(1R,2R)-2-Hydroxy-5-(3-methylaminopropyl)cyclohexylsulfanyl]-acetyl}-mutilin und dem (1S,2S)-Diastereomer davon
14-O-{[(1R,2R)-2-Hydroxy-4-(3-methylaminopropyl)-cyclohexylsulfanyl]-acetyl}-mutilin und dem (1S,2S)-Diastereomer davon
14-O-{[(1R,2R)-5-(3-Aminopropyl)-2-hydroxycyclohexylsulfanyl]acetyl}-mutilin und dem (1S,2S)-Diastereomer davon
14-O-{[(1R,2R)-4-(3-Aminopropyl)-2-hydroxycyclohexylsulfanyl]acetyl}-mutilin und dem (1S,2S)-Diastereomer davon
14-O-{[(6R,8R)-8-Amino-1,4-dioxa-spiro[4.5]dec-6-ylsulfanyl]acetyl}-mutilin und dem (6S,8S)-Diastereomer davon
14-O-{[4-Amino-2-methoxy-cyclohexylsulfanyl]-acetyl}-mutilin
und
14-O-{[5-Amino-2-methoxy-cyclohexylsulfanyl]-acetyl}-mutilin.

4. Verbindung zur Verwendung gemäß einem der Ansprüche 1 bis 3, wobei es sich bei der Verbindung um Lefamulin handelt.

5. Verbindung zur Verwendung gemäß einem der Ansprüche 1 bis 4, wobei die Verbindung in Form eines Salzes und/oder eines Solvats vorliegt.

6. Verbindung zur Verwendung gemäß einem der Ansprüche 1 bis 5, wobei es sich bei der Verbindung um Lefamulin in Form von Lefamulin-Acetatsalz handelt.

7. Verbindung zur Verwendung gemäß einem der vorhergehenden Ansprüche, wobei die bakterielle Infektion durch Bakterien vermittelt wird, die aus dem Stamm der Spirochaetes ausgewählt sind.

8. Verbindung zur Verwendung gemäß Anspruch 7, wobei die Bakterien aus der Ordnung der Spirochaetales oder Leptospirales ausgewählt sind.

9. Verbindung zur Verwendung gemäß Anspruch 7 oder 8, wobei die Bakterien aus der Ordnung der Spirochaetales ausgewählt sind.

10. Verbindung zur Verwendung gemäß einem der Ansprüche 1 bis 8, wobei die Bakterien ausgewählt sind aus der Gruppe, bestehend aus den Gattungen *Borrelia*, *Borreliella*, *Leptospira* und *Treponema*, vorzugsweise aus der Gruppe, bestehend aus den Gattungen *Borreliella* und *Treponema*.

11. Verbindung zur Verwendung gemäß einem der Ansprüche 1 bis 10, wobei die bakterielle Infektion durch *Treponema* vermittelt wird.

12. Verbindung zur Verwendung gemäß Anspruch 11, wobei die bakterielle Infektion aus der Gruppe von Syphilis, einschließlich venerischer und endemischer Syphilis, Pinta, (treponemaler) Parodontitis und Frambösie, vorzugsweise Syphilis, ausgewählt ist.

13. Verbindung zur Verwendung gemäß einem der Ansprüche 1 bis 9, wobei die bakterielle Infektion durch Bakterien der Familie der Borreliaceae, vorzugsweise durch *Borreliella* oder *Borrelia*, stärker bevorzugt *Borreliella*, vermittelt wird.

14. Verbindung zur Verwendung gemäß Anspruch 13, wobei die bakterielle Infektion aus der Gruppe von Lyme-Krankheit und Rückfallfieber, vorzugsweise Lyme-Krankheit (einschließlich Lyme-Borreliose), ausgewählt ist.

15. Verbindung zur Verwendung gemäß einem der vorhergehenden Ansprüche, wobei die Verbindung einem Menschen verabreicht wird.

## Revendications

1. Composé de formule (I) 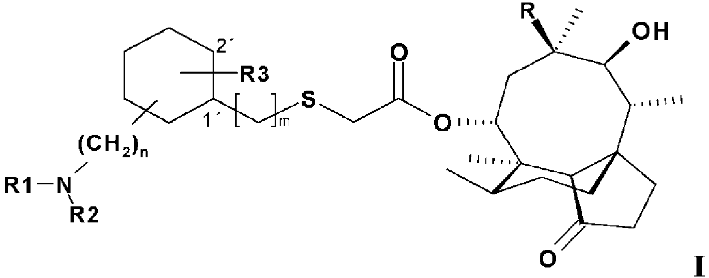 dans lequel
n vaut 0 à 4;
m vaut 0 ou 1 à condition que l'atome du soufre et R₃ soient en position vicinale (si m = 0 alors R₃ est en position 2', et si m = 1 alors R₃ est sur la position 1');
R est un éthyle ou un vinyle;
R₁ est un hydrogène ou un alkyle en (C₁₋₆),
R₂ est un hydrogène ou
- un cycloalkyle en (C₃₋₆), ou
- un alkyle en (C₁₋₆) non substitué, ou
- un alkyle en (C₁₋₆) substitué par un ou plusieurs parmi
- un hydroxy; de préférence un ou deux,
- un méthoxy,
- un halogène, et
- un cycloalkyle en (C₃₋₆), ou
R₁ et R₂ conjointement à l'atome d'azote auquel ils sont fixés forment un cycle hétérocyclique de 5 à 7 chaînons contenant au moins 1 atome d'azote ou 1 azote et 1 hétéroatome supplémentaire choisi par exemple parmi N ou O, ou
R₁ est un hydroxy et R₂ est un formyle;
R₃ est OH, OR₄, un atome d'halogène, ou
R₃ est lié à 2', et représente -O-(CH₂)ₚ-O- avec p vaut 2 ou 3;
R₄ est un alkyle en (C₁₋₆) non substitué ou un cycloalkyle en (C₃₋₆),
ou un sel ou solvate pharmaceutiquement acceptable de celui-ci,
pour l'utilisation spécifique dans le traitement ou la prévention d'une infection bactérienne médiée par des spirochètes.

2. Composé destiné à être utilisé selon la revendication 1, dans lequel le composé est choisi dans le groupe constitué par les composés de formule (II), (III), (IV), (V) et (VI) 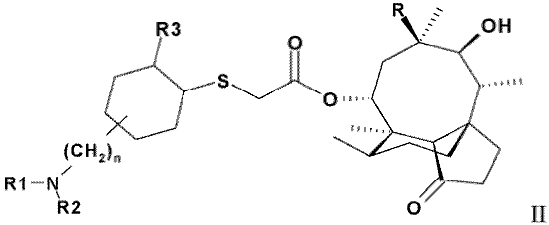 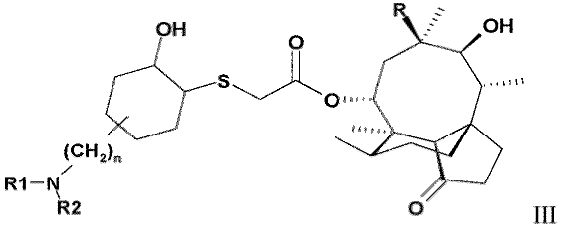 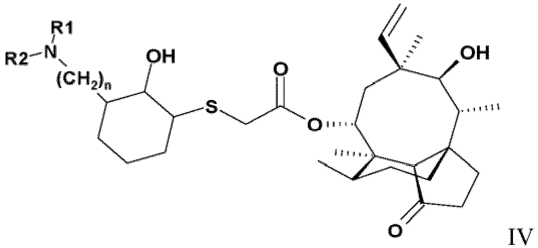 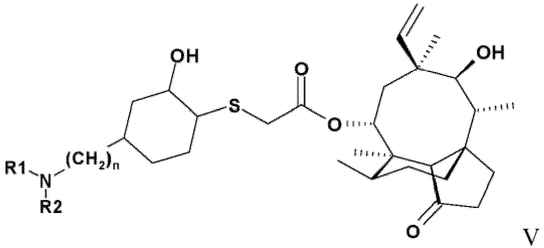 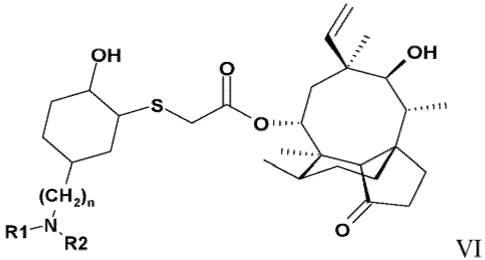 dans lequel n, R₁ et R₂ sont chacun tels que définis dans la revendication 1.

3. Composé destiné à être utilisé selon la revendication 1 ou 2, dans lequel le composé est choisi dans le groupe constitué par
la 14-O-{[(1R,2R,4R)-4-amino-2-hydroxy-cyclohexylsulfanyl]-acétyl}-mutiline
la 14-O-{[(1S,2S,4S)-4-amino-2-hydroxy-cyclohexylsulfanyl]-acétyl}-mutiline
la 14-O-{[(1R,2R,5S)-5-amino-2-hydroxy-cyclohexylsulfanyl]-acétyl}-mutiline
la 14-O-{[(1S,2S,5R)-5-amino-2-hydroxy-cyclohexylsulfanyl]-acétyl}-mutiline
la 14-O-{[(1R,2R,4S)-4-amino-2-hydroxy-cyclohexylsulfanyl]-acétyl}-mutiline et le diastéréomère (1S,2S,4R) de celle-ci
la 14-O-{[(1R,2R,5R)-5-amino-2-hydroxy-cyclohexylsulfanyl]-acétyl}-mutiline
la 14-O-{[(1S,2S,5S)-5-amino-2-hydroxy-cyclohexylsulfanyl]-acétyl}-mutiline
la 14-O-{[(1R,2R,3R)-3-amino-2-hydroxy-cyclohexylsulfanyl]-acétyl}-mutiline et le diastéréomère (1S,2S,3S) de celle-ci
la 14-O-{[(1R,2R,4R)-4-diéthylamino-2-hydroxy-cyclohexylsulfanyl]-acétyl}-mutiline et le diastéréomère (1S,2S,4S) de celle-ci
la 14-O-{[(1R,2R,4R)-4-éthylamino-2-hydroxy-cyclohexylsulfanyl]-acétyl}-mutiline et le diastéréomère (1S,2S,4S) de celle-ci
la 14-O-{[(1R,2R,5S)-5-éthylamino-2-hydroxy-cyclohexylsulfanyl]-acétyl}-mutiline et le diastéréomère (1S,2S,5R) de celle-ci
la 14-O-{[(1R,2R,5S)-5-diéthylamino-2-hydroxy-cyclohexylsulfanyl]-acétyl}-mutiline et le diastéréomère (1S,2S,5R) de celle-ci
la 14-O-{[(1R,2R,4S)-4-diéthylamino-2-hydroxy-cyclohexylsulfanyl]-acétyl}-mutiline et le diastéréomère (1S,2S,4R) de celle-ci
la 14-O-{[(1R,2R,5R)-5-diéthylamino-2-hydroxy-cyclohexylsulfanyl]-acétyl}-mutiline et le diastéréomère (1S,2S,5S) de celle-ci
la 14-O-{[(1R,2R,3R)-3-éthylamino-2-hydroxy-cyclohexylsulfanyl]-acétyl}-mutiline et le diastéréomère (1S,2S,3S) de celle-ci
la 14-O-{[(1R,2R,3R)-3-diéthylamino-2-hydroxy-cyclohexylsulfanyl]-acétyl}-mutiline et le diastéréomère (1S,2S,3S) de celle-ci
la 14-O-{[(1R,2R,4S)-4-(formyl-hydroxy-amino)-2-hydroxy-cyclohexylsulfanyl]-acétyl}-mutiline et le diastéréomère (1S,2S,4R) de celle-ci
la 14-O-{[(1R,2R,5S)-5-(formyl-hydroxy-amino)-2-hydroxy-cyclohexylsulfanyl]-acétyl}-mutiline et le diastéréomère (1S,2S,5R) de celle-ci
la 14-O-{[(1R,2R,3R/S)-3-(formyl-hydroxy-amino)-2-hydroxy-cyclohexylsulfanyl]-acétyl}-mutiline et le diastéréomère (1S,2S,3R/S) de celle-ci
la 14-O-{[(1R,2R,5S)-2-hydroxy-5-méthylamino-cyclohexylsulfanyl]-acétyl}-mutiline et le diastéréomère (1S,2S,5R) de celle-ci
la 14-O-{[(1R,2R,5S)-5-allylamino-2-hydroxy-cyclohexylsulfanyl]-acétyl}-mutiline et le diastéréomère (1S,2S,5R) de celle-ci
la 14-O-{[(1R,2R,5S)-2-hydroxy-5-(2-méthoxy-éthylamino)-cyclohexylsulfanyl]-acétyl}-mutiline et le diastéréomère (1S,2S,5R) de celle-ci
la 14-O-{[(1R,2R,4R*)-2-hydroxy-4-(2-hydroxy-éthylamino)-cyclohexylsulfanyl]-acétyl}-mutiline et le diastéréomère (1S,2S,4S*) de celle-ci
la 14-O-{[(1R,2R,4R*)-4-cyclohexylamino-2-hydroxy-cyclohexylsulfanyl]-acétyl}-mutiline et le diastéréomère (1S,2S,4S*) de celle-ci
la 14-O-{[(1R,2R,4R*)-4-cyclopropylamino-2-hydroxy-cyclohexylsulfanyl]-acétyl}-mutiline et le diastéréomère (1S,2S,4S*) de celle-ci
la 14-O-{[(1R,2R,5S*)-4-cyclopropylamino-2-hydroxy-cyclohexylsulfanyl]-acétyl}-mutiline et le diastéréomère (1S,2S,5R*) de celle-ci
la 14-O-{[(1R,2R,4S*)-4-cyclopropylamino-2-hydroxy-cyclohexylsulfanyl]-acétyl}-mutiline et le diastéréomère (1S,2S,4R*) de celle-ci
la 14-O-{[(1R,2R,5R*)-2-hydroxy-5-morpholin-4-yl-cyclohexylsulfanyl]-acétyl}-mutiline et le diastéréomère (1S,2S,5S*) de celle-ci
la 14-O-{[(1R,2R,5S*)-2-hydroxy-5-morpholin-4-yl-cyclohexylsulfanyl]-acétyl}-mutiline et le diastéréomère (1S,2S,5R*) de celle-ci
la 14-O-{[(1R,2R,5S)-5-amino-2-hydroxy-cyclohexylsulfanyl]-acétyl}-19,20-dihydro-mutiline et le diastéréomère (1S,2S,5R) de celle-ci
la 14-O-{[(1R,2R,5S)-5-éthylamino-2-hydroxy-cyclohexylsulfanyl]-acétyl}-19,20-dihydro-mutiline et le diastéréomère (1S,2S,5R) de celle-ci
la 14-O-{[(1R,2R,5R)-5-amino-2-hydroxy-cyclohexylsulfanyl]-acétyl}-19,20-dihydro-mutiline et le diastéréomère (1S,2S,5S) de celle-ci
la 14-O-{[(1R,2R)-4-aminométhyl-2-hydroxy-cyclohexylsulfanyl]-acétyl}-mutiline et les diastéréomères (1S,2S) de celle-ci
la 14-O-{[5-amino-2-chloro-cyclohexylsulfanyl]-acétyl}-mutiline
la 14-O-{[4-amino-2-chloro-cyclohexylsulfanyl]-acétyl}-mutiline
la 14-O-[(4-amino-1-hydroxy-cyclohexylméthylsulfanyl)-acétyl]-mutiline
la 14-O-{[(1R,2R)-2-hydroxy-5-(3-méthylamino-propyl)-cyclohexylsulfanyl]-acétyl}-mutiline et le diastéréomère (1S,2S) de celle-ci
la 14-O-{[(1R,2R)-2-hydroxy-4-(3-méthylamino-propyl)-cyclohexylsulfanyl]-acétyl}-mutiline et le diastéréomère (1S,2S) de celle-ci
la 14-O-{[(1R,2R)-5-(3-amino-propyl)-2-hydroxy-cyclohexylsulfanyl]-acétyl}-mutiline et le diastéréomère (1S,2S) de celle-ci
la 14-O-{[(1R,2R)-4-(3-amino-propyl)-2-hydroxy-cyclohexylsulfanyl]-acétyl}-mutiline et le diastéréomère (1S,2S) de celle-ci
la 14-O-{[(6R,8R)-8-amino-1,4-dioxa-spiro[4.5]déc-6-ylsulfanyl]-acétyl}-mutiline et le diastéréomère (6S,8S) de celle-ci
la 14-O-{[4-amino-2-méthoxy-cyclohexylsulfanyl]-acétyl}-mutiline
et
la 14-O-{[5-amino-2-méthoxy-cyclohexylsulfanyl]-acétyl}-mutiline.

4. Composé destiné à être utilisé selon l'une quelconque des revendications 1 à 3, dans lequel le composé est la léfamuline.

5. Composé destiné à être utilisé selon l'une quelconque des revendications 1 à 4, dans lequel le composé se présente sous forme de sel et/ou de solvate.

6. Composé destiné à être utilisé selon l'une quelconque des revendications 1 à 5, dans lequel le composé est la léfamuline sous forme de sel d'acétate de léfamuline.

7. Composé destiné à être utilisé selon l'une quelconque des revendications précédentes, dans lequel l'infection bactérienne est médiée par des bactéries sélectionnées parmi le phylum des Spirochaetes.

8. Composé destiné à être utilisé selon la revendication 7, dans lequel les bactéries sont sélectionnées parmi l'ordre des Spirochaetales ou des Leptospirales.

9. Composé destiné à être utilisé selon la revendication 7 ou 8, dans lequel les bactéries sont sélectionnées parmi l'ordre des Spirochaetales.

10. Composé destiné à être utilisé selon l'une quelconque des revendications 1 à 8, dans lequel les bactéries sont sélectionnées dans le groupe constitué par les genres *Borrelia*, *Borreliella*, *Leptospira* et *Treponema*, de préférence dans le groupe constitué par les genres *Borreliella* et *Treponema*.

11. Composé destiné à être utilisé selon l'une quelconque des revendications 1 à 10, dans lequel l'infection bactérienne est médiée par *Treponema*.

12. Composé destiné à être utilisé selon la revendication 11, dans lequel l'infection bactérienne est sélectionnée dans le groupe comprenant la syphilis, y compris la syphilis vénérienne et endémique, la pinta, la parodontite (tréponémique) et le pian, de préférence la syphilis.

13. Composé destiné à être utilisé selon l'une quelconque des revendications 1 à 9, dans lequel l'infection bactérienne est médiée par des bactéries de la famille des Borreliaceae, de préférence par *Borreliella* ou *Borrelia*, plus préférentiellement par *Borreliella*.

14. Composé destiné à être utilisé selon la revendication 13, dans lequel l'infection bactérienne est sélectionnée dans le groupe comprenant la maladie de Lyme et la fièvre récurrente, de préférence la maladie de Lyme (y compris la borréliose de Lyme).

15. Composé destiné à être utilisé selon l'une quelconque des revendications précédentes, dans lequel le composé est administré à un être humain.
